Europäisches Patentamt

European Patent Office

Office européen des brevets

(19)

(11) **EP 0 706 572 B1**

(12) **EUROPEAN PATENT SPECIFICATION**

(45) Date of publication and mention
of the grant of the patent:
**19.01.2005 Bulletin 2005/03**

(21) Application number: **94923229.2**

(22) Date of filing: **29.06.1994**

(51) Int Cl.[7]: **C12N 15/53**, C12N 9/04,
C12N 9/08, C12N 15/81,
C12N 1/19
// C12N1:19, C12R1:78

(86) International application number:
**PCT/US1994/007080**

(87) International publication number:
**WO 1995/001443 (12.01.1995 Gazette 1995/03)**

(54) **MICROBIAL TRANSFORMANTS OF HANSENULA THAT EXPRESS GLYCOLATE OXYDASE AND CATALASE**

MIKROBIELLE, TRANSFORMIERTE HANSENULAZELLEN, WELCHE GLYCOLAT-OXIDASE UND KATALASE EXPRIMIEREN

TRANSFORMANTS MICROBIENS D'HANSENULA EXPRIMANT L'OXYDASE DE GLYCOLATE ET LA CATALASE

(84) Designated Contracting States:
**FR GB IT**

(30) Priority: **01.07.1993 US 85491**

(43) Date of publication of application:
**17.04.1996 Bulletin 1996/16**

(73) Proprietor: **University of Iowa Research
Foundation
Iowa City, IA 52242-5000 (US)**

(72) Inventors:
• **ANTON, David, Leroy
Wilmington, DE 19803 (US)**
• **DiCOSIMO, Robert
Wilmington, DE 19808 (US)**
• **DAHLEMS, Ulrike, Magdalene
D-47807 Krefeld (DE)**
• **GELLISSEN, Gerd, Johann
D-42489 Wulfrath (DE)**
• **JONOWICZ, Zbigniew, Alojzy
D-4006 Erkrath (DE)**
• **PIONTEK, Michael
D-45259 Essen (DE)**

(74) Representative: **Grant, Anne Rosemary et al
Frank B. Dehn & Co.
179 Queen Victoria Street
London EC4V 4EL (GB)**

(56) References cited:
WO-A-93/09242      WO-A-93/09243
WO-A-93/14214

• **BIOCHEMISTRY., vol.30, no.18, 7 May 1991,
EASTON, PA US pages 4612 - 4619 PETER
MACHEROUX ET AL. 'Expression of spinach
glycolate oxidase in Saccharomyces cerevisiae:
Purification and characterization.' cited in the
application**
• **JOURNAL OF BIOLOGICAL CHEMISTRY,
vol.262, no.33, 25 November 1987, BALTIMORE,
MD US pages 15825 - 15828 MICHA VOLOKITA
ET AL. 'The primary structure of spinach
glycolate oxidase deduced from the DNA
sequence of a cDNA clone.' cited in the
application**
• **CHEMICAL ABSTRACTS, vol. 112, no. 13, 26
March 1990, Columbus, Ohio, US; abstract no.
113606b, TANAKA, ATSUO ET AL. page 239 ; &
JP,A,01 086 879 (MITSUBISHI) 31 March 1989**
• **CHEMICAL ABSTRACTS, vol. 109, no. 7, 15
August 1988, Columbus, Ohio, US; abstract no.
49626s, TAKAHASHI, MASASHI page 200 ; &
JP,A,63 017 693 (MITSUI TOATSU) 25 January
1988**

- **EUROPEAN JOURNAL OF BIOCHEMISTRY, vol.184, no.1, September 1989 pages 173 - 179 H. HANSEN ET AL. 'Functional complementation of catalase-defective peroxisomes in a methylotrophic yeast by import of the catalase A from Saccharomyces cerevisiae' cited in the application**

SEQUENCE LISTING

(1) GENERAL INFORMATION:

(i) APPLICANT:

(A) NAME: E. I. DU PONT DE NEMOURS AND COMPANY
(B) STREET: 1007 MARKET STREET
(C) CITY: WILMINGTON
(D) STATE: DELAWARE
(E) COUNTRY: UNITED STATES OF AMERICA
(F) POSTAL CODE (ZIP): 19898
(G) TELEPHONE: 302-992-4929
(H) TELEFAX: 302-773-0164
(I) TELEX: 6717325

(ii) TITLE OF INVENTION: MICROBIAL TRANSFORMANTS OF *HANSENULA* THAT EXPRESS GLYCOLATE OXIDASE AND CATALASE

(iii) NUMBER OF SEQUENCES: 1

(iv) COMPUTER READABLE FORM:

(A) MEDIUM TYPE: FLOPPY DISK
(B) COMPUTER: IBM
(C) OPERATING SYSTEM: MICROSOFT WINDOWS V3.0
(D) SOFTWARE: MICROSOFT WORD V2.0C

(v) CURRENT APPLICATION DATA:

(A) APPLICATION NUMBER:

(vi) PRIOR APPLICATION DATA:

(A) APPLICATION NUMBER: 08/085,491
(B) FILING DATE: JULY 1, 1993

(2) INFORMATION FOR SEQ ID NO: 1:

(i) SEQUENCE CHARACTERISTICS:

(A) LENGTH: 369 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:1:

```
Met Glu Ile Thr Asn Val Asn Glu Tyr Glu Ala Ile Ala Lys Gln Lys
1               5                   10                  15

Leu Pro Lys Met Val Tyr Asp Tyr Tyr Ala Ser Gly Ala Glu Asp Gln
            20              25                  30

Trp Thr Leu Ala Glu Asn Arg Asn Ala Phe Ser Arg Ile Leu Phe Arg
            35              40                  45

Pro Arg Ile Leu Ile Asp Val Thr Asn Ile Asp Met Thr Thr Thr Ile
        50              55                  60

Leu Gly Phe Lys Ile Ser Met Pro Ile Met Ile Ala Pro Thr Ala Met
65              70                  75                  80

Gln Lys Met Ala His Pro Glu Gly Glu Tyr Ala Thr Ala Arg Ala Ala
                85              90                  95

Ser Ala Ala Gly Thr Ile Met Thr Leu Ser Ser Trp Ala Thr Ser Ser
            100             105                 110

Val Glu Glu Val Ala Ser Thr Gly Pro Gly Ile Arg Phe Phe Gln Leu
            115             120                 125

Tyr Val Tyr Lys Asp Arg Asn Val Val Ala Gln Leu Val Arg Arg Ala
        130             135                 140

Glu Arg Ala Gly Phe Lys Ala Ile Ala Leu Thr Val Asp Thr Pro Arg
145             150                 155                 160

Leu Gly Arg Arg Glu Ala Asp Ile Lys Asn Arg Phe Val Leu Pro Pro
            165             170                 175

Phe Leu Thr Leu Lys Asn Phe Glu Gly Ile Asp Leu Gly Lys Met Asp
            180             185                 190

Lys Ala Asn Asp Ser Gly Leu Ser Ser Tyr Val Ala Gly Gln Ile Asp
            195             200                 205

Arg Ser Leu Ser Trp Lys Asp Val Ala Trp Leu Gln Thr Ile Thr Ser
    210             215                 220
```

```
Leu Pro Ile Leu Val Lys Gly Val Ile Thr Ala Glu Asp Ala Arg Leu
225             230             235             240

Ala Val Gln His Gly Ala Ala Gly Ile Ile Val Ser Asn His Gly Ala
                245             250             255

Arg Gln Leu Asp Tyr Val Pro Ala Thr Ile Met Ala Leu Glu Glu Val
            260             265             270

Val Lys Ala Ala Gln Gly Arg Ile Pro Val Phe Leu Asp Gly Gly Val
            275             280             285

Arg Arg Gly Thr Asp Val Phe Lys Ala Leu Ala Leu Gly Ala Ala Gly
    290             295             300             .

Val Phe Ile Gly Arg Pro Val Val Phe Ser Leu Ala Ala Glu Gly Glu
305             310             315             320

Ala Gly Val Lys Lys Val Leu Gln Met Met Arg Asp Glu Phe Glu Leu
            325             330             335

Thr Met Ala Leu Ser Gly Cys Arg Ser Leu Lys Glu Ile Ser Arg Ser
            340             345             350

His Ile Ala Ala Asp Trp Asp Gly Pro Ser Ser Arg Ala Val Ala Arg
            355             360             365

Leu
```

**Description**

1. Field of Invention:

The present invention relates to novel microbial transformants that express enzymatically active glycolate oxidase. More specifically, the invention relates to culturing a transformant of *Hansenula* methylotrophic yeast species comprising a heterologous DNA encoding glycolate oxidase and optionally transformed with DNA encoding an exogenous catalase for the production of enzymatically active glycolate oxidase and, optionally, an enzymatically active heterologous catalase.

2. Description of the Related Art:

Glyoxylic acid is an important intermediate in the manufacture of various agrochemicals, pharmaceuticals and fragrances. Typical commercial production of glyoxylic acid employs either oxidation chemistry or electrochemical means. Electrochemical manufacture involves either the reduction of oxalic acid or the anodic oxidation of glyoxal to form glyoxylic acid, whereas chemical oxidation generally involves the oxidation of glyoxal in the presence of a strong acid such as $HNO_3$. A consequence of these commercial processes is the production of waste streams containing various toxic acids and heavy metals. Increased public concern coupled with mounting government regulations surrounding the production of toxic wastes has provided impetus for a search for an alternative, cost effective, yet environmentally acceptable method of glyoxylic acid production. One such potential route lies in microbially mediated enzymatic catalysis involving the oxidation of glycolic acid by glycolate oxidase such as disclosed in U.S. Patent 5,219,745. Glycolic acid and oxygen were reacted in an aqueous solution and in the presence of an amine buffer and two enzyme catalysts, glycolate oxidase and catalase. This process demonstrated the synergistic effect of using both catalase (to destroy byproduct hydrogen peroxide responsible for formate production) and an amine additive capable of forming oxidation-resistant N-substimred hemiaminal and/or imine complexes with glyoxylate (limiting its further oxidation). A related process for the preparation of mixtures of glyoxylic acid and aminomethylphosphonic acid (AMPA) has been described in U.S. Patent 5,135,860. In U.S. Patent 5,180,846, the resulting mixtures of glyoxylic acid and AMPA were used for the preparation of N-(phosphonomethyl)glycine, a post-emergent phytotoxicant and herbicide.

Glycolate oxidase is an enzyme available from various sources, including green leafy plants and mammalian cells. N. E. Tolbert et al. (J. Biol. Chem., (1949) 181:905) first reported an enzyme, extracted from tobacco leaves, which catalyzed the oxidation of glycolic acid to formic acid and $CO_2$ via the intermediate formation of glyoxylic acid. Similarly, K. E. Richardson and N. E. Tolbert (J. Biol. Chem., (1961) 236:1280) reported the formation of oxalic acid during the glycolate oxidase-catalyzed oxidation of glycolic acid to glyoxylic acid, using enzymes isolated from tobacco, sugar beet, Swiss chard, spinach, or rat liver. C. O. Clagett, N. E. Tolbert and R. H. Burris (J. Biol. Chem., (1949) 78: 977) reported that the optimum pH for the glycolate oxidase catalyzed oxidation of glycolic acid with oxygen was about 7.8-8.6, and the optimum temperature was 35-40 °C.

Recent advances in recombinant DNA technology, combined with the isolation of cDNA coding for the spinach enzyme (see Volokita et al., J. Biol. Chem., (1987) 262(33):15825) have allowed for the construction of microbial strains that are intended to serve as alternative, economic enzyme sources. To date, the host organism of choice for expression of heterologous proteins in commercial applications has been *Escherichia coli.* However, in some situations *Escherichia coli* may prove to be an unsuitable host. For example, *Escherichia coli* contains a number of proteolytic factors that may interfere with the activity of the expressed enzyme and may also produce toxic pyrogenic factors requiring further purification. These and other considerations have led to increased interest in alternative hosts, in particular yeast, for the production of active enzymes.

Yeast offer several advantages to commercial applications over *Escherichia coli* and other bacteria. Yeast can generally be grown to higher densities than bacteria and are readily adaptable to continuous fermentation processing. Additional advantages of yeast hosts include the fact that many critical functions of the organism, such as oxidative phosphorylations, are located within organelles and thus are not exposed to the possible deleterious effects of the organism's over-expression of foreign enzymatic products. Furthermore, yeast appear to be capable of glycosylation of expressed polypeptide products where such glycosylation is important to the bioactivity of the polypeptide product.

Despite having the genetic capacity to produce glycolate oxidase, the typical microbial hosts used to date have performed poorly, producing either inactive enzyme, or producing the enzyme at very modest levels. Although early data indicated that *Escherichia coli* was unable to produce glycolate oxidase in enzymatically active form following introduction of the enzyme-encoding gene in expressible form (see Macheroux et al., Biochemistry (1991) 30: 4612), it has since been determined that active enzyme could indeed be expressed in this host using a T7 RNA polymerase promoter, albeit in very modest amounts (Macheroux et al., Biochem. Biophys., (1992), Acta.,1132: 11-16). It has been possible to achieve expression of active glycolate oxidase from various species of fungi including those of the genus *Saccharomyces* (Macheroux et al., *supra* (1991)); however, these hosts have only been capable of producing enzymes at levels far below what would be considered commercially useful.

Catalase (e.g., EC 1.11.1.6) is an enzyme widely distributed in nature, and found in virtually all aerobic organisms. It functions to disproportionate hydrogen peroxide formed either by the reaction of oxidase enzymes or by reactxoas of molecular oxygen. The reaction is as follows:

$$2H_2O_2 \rightarrow 2H_2O + O_2$$

In eukaryotic organisms the catalase is often present in at least two different forms: cytosolic, and peroxisomal; for example, in *Saccharomyces cerevisiae* these two forms are catalase T (CTT1 gene product, Spevak, W., et al., Mol. Cell Biol, (1983), 3:1545) and catalase A (CTA1 gene product, Cohen et al., Mol. Gen. Genet., (1985), 200:74-79), respective Catalase A and catalase T have been reported to be actively expressed in *Hansenula polymorpha* under the control of their native *Saccharomyces cerevisiae* promoters, however, only catalase A has been found to functionally complement catalase-defective peroxisomes (Hansen and Roggenkamp, Eur. J. Biochem., (1989), 184:173-179).

SUMMARY OF THE INVENTION

The present invention provides a transformed *Hansenula* host yeast for the expression of the enzymatically active glycolate oxidase wherein the transformed *Hansenula* host yeast comprises a heterologous DNA fusion capable of expressing glycolate oxidase. Preferably the transformed *Hansenula* host yeast is also capable of expressing an exogenous catalase gene.

In one specific embodiment of the invention, a doubly transformed *Hansenula polymorpha* is provided which co-expresses enzymatically active, heterologously produced catalase along with the enzymatically active glycolate oxidase. Preferably, the doubly transformed *Hansenula* host yeast is capable of expressing an exogenously derived catalase gene selected so as not to be inhibited by aminomemylphosphonic acid. A further specific embodiment uses heterologous DNA that encodes for spinach glycolate oxidase or an enzymatically active variant thereof. In all embodiments the heterologous DNA is stably integrated within the host chromosome.

The present invention also provides a process for the production of enzymatically active glycolate oxidase comprising the steps of culturing a transformed *Hansenula* strain comprising a heterologous DNA construct encoding enzymatically active glycolate oxidase and maintaining the growth of said yeast under conditions suitable for the maximal production of glycolate oxidase. Optionally, a method is also provided where exogenous catalase is co-expressed with said glycolate oxidase.

BRIEF DESCRIPTION OF THE DRAWINGS,

SEQUENCE LISTING AND BIOLOGICAL DEPOSITS

Figure 1 illustrates the amino acid sequence of spinach glycolate oxidase (SEQ ID NO.:1)
Figure 2 illustrates the plasmid pFPMT130 expression vector indicating the respective components and restriction sites.
Figure 3 illustrates the plasmid pRB expression vector indicating the respective components and restriction sites.
Figure 4 illustrates the plasmid pFMDGO expression vector indicating the respective components and restriction sites.
Figure 5 illustrates the plasmid pRBGO expression vector indicating the respective components and restriction sites.
Figure 6 illustrates the plasmid pHK2 expression vector indicating the respective components and restriction sites.
Figure 7 illustrates the plasmid pHCTKm2 expression vector indicating the respective components and restriction sites.

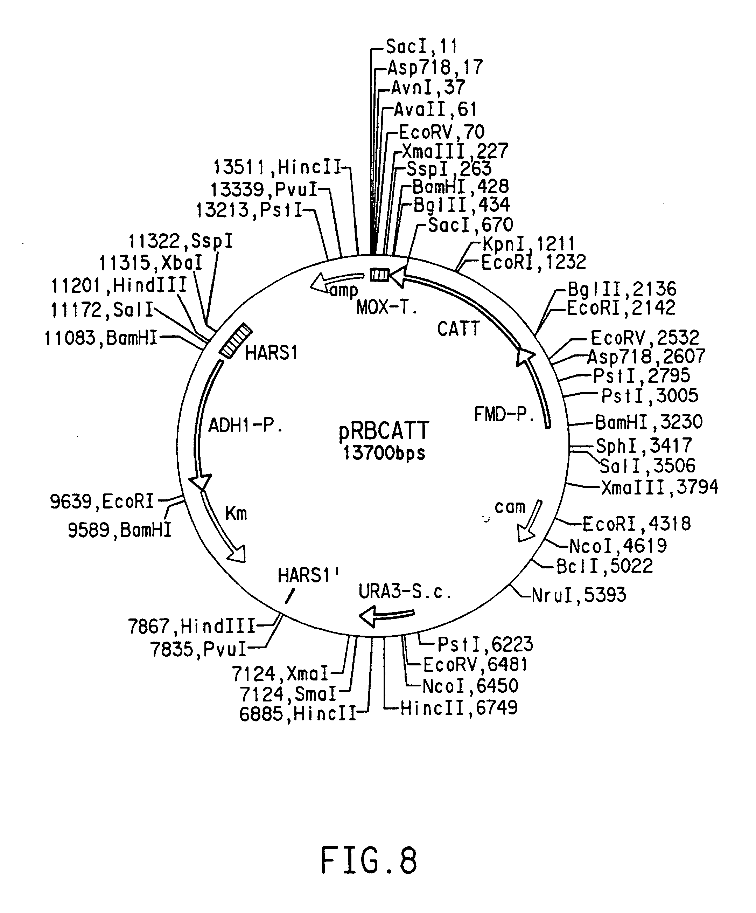

Figure 8 illustrates the plasmid pRBCATT expression vector indicating the respective components and restriction sites.

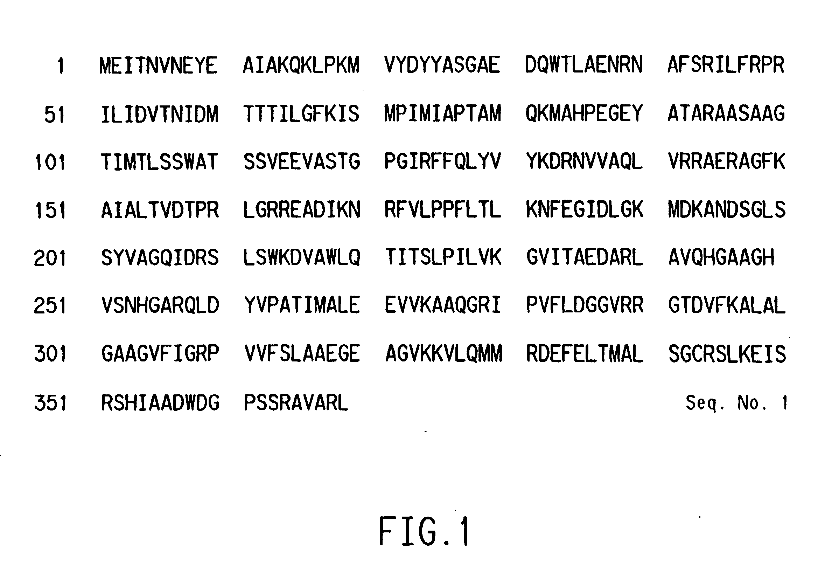

Applicants have provided a sequence listing for SEQ ID NO.:1 in conformity with "Rules for the Standard Representation of Nucleotide and Amino Acid Sequences in Patent Applications" (Annexes I and II to the Decision of the President of the EPO, published in Supplement No. 2 to OJ EPO 12/1992). This sequence corresponds to Figure 1.

Applicants have made the following biological deposits under the terms of the Budapest Treaty:

| Depositor's Identification Reference | NRRL Designation | Deposit Date |
|---|---|---|
| *Hansenula polymorpha,* G01 | NRRL Y-21065 | 30 March 1993 |
| *Hansenula polymorpha,* G02 | NRRL Y-21113 | 25 June 1993 |
| *Hansenula polymorpha,* G03 | NRRL Y-21114 | 25 June 1993 |

As used herein, the designation "NRRL" refers to the Northern Regional Research Laboratories, U.S. Department of Agriculture, Agricultural Research Service Culture Collection, 1815 N. University Street, Peoria, IL 61604, U.S.A.

DETAILED DESCRIPTION OF THE INVENTION

The present invention relates to the production of enzymatically active glycolate oxidase (GO) by yeast of the genus *Hansenula* in commercially useful quantities. In using the term *"Hansenula* strain" it should be understood that term encompasses any species that shares the same or similar taxonomy of a methyloltropic *Hansenula* yeast and as such it is felt that the designation of the host as a species of the genus *Hansenula* is not controlling but rather the methyloltropic yeast taxonomy is a more appropriate criteria. This would include by way of example but not limited thereto such species as *Hansenula polymorpha, Hansenula augusta, Hansenula anomala, Hansenula capsulata, Hansenula saturnus,* and *Hansenula wingei.* For purpose of this invention it should also be appreciated that the phrase "enzymatically active glycolate oxidase" refers to any glycolate oxidase in a form capable of converting glycolate to glyoxylate (i.e., converting glycolic acid to glyoxylic acid) as determined by assays of conventional design. It should be further understood that the phrase also encompasses enzyme contained within vesicles such as inclusion bodies where the enzyme must be isolated and refolded in order to catalyze the glycolate to glyoxylate reaction. Further, for purposes of this invention, it is understood that "commercially useful quantities" refers to production of the specific proteins in concentrations per unit of cells that are economically feasible.

Thus the glycolate oxidase of the present invention may have a structure corresponding to any naturally occurring form of the enzyme, or may have a genetically engineered variant structure, provided, however, that enzymatically active glycolate oxidase, as defined above, is retained. Naturally occurring forms of glycolate oxidase include, for example, spinach-produced glycolate oxidase. As shown in Figure 1 herein, spinach glycolate oxidase consists, in its mature form, of 369 amino acids arranged in the indicated sequence (SEQ ID NO.:1). According to a preferred embodiment of the present invention, the glycolate oxidase is spinach glycolate oxidase or an enzymatically active variant of spinach glycolate oxidase, e.g. an enzymatically active fragment of the enzyme, or an analog in which one or more amino acids is replaced using conservative amino acid replacements, or a variant in which the region of the enzyme which directs its peroxisomal accumulation is deleted (see for example Macheroux et al., *supra* (1991)).

The catalase from *Aspergillus niger, Saccharomyces cerevisiae* (catalase *T), Hansenula polymorpha, Pichia pastoris,* and bovine liver have now each been examined as catalyst for the decomposition of hydrogen peroxide generated during the oxidation of glycolic acid/AMPA mixtures, and a previously-unreported, reversible inhibition of the catalase from *H. polymorpha, P. pastoris,* and bovine liver by AMPA has been discovered. This inhibition occurs regardless of whether a soluble catalase, immobilized catalase, or catalase-containing whole cell catalyst is employed.

As an alternative to adding an additional source of inhibition-resistant catalase to glycolic acid/AMPA reaction mixtures which use as catalyst a microbial cell transformant whose endogenous catalase is inhibited by AMPA (e.g., *H. polymorpha* transformants), the microbial transformant (which expresses the exogenous enzyme glycolate oxidase) has been re-engineered to express an additional exogenous catalase (e.g., catalase T from S. *cerevisiae)* which is not inhibited by AMPA. Using this microbial cell "double" transformant as catalyst in the absence of an added catalase which is not inhibited by AMPA has resulted in a significant increase in glyoxylate yield and reduction in formate production when compared to that obtained with a microbial cell "single" transformant (i.e., exogenous glycolate oxidase and an endogenous catalase) whose catalase is inhibited by AMPA. It should be appreciated that for enzymatic conversion of glycolate to glyoxylate in the presence of AMPA the heterologous catalase is to be chosen by virtue of its lack of inhibition by AMPA. These include by way of example but are not limited to the catalase T from *Saccharomyces cerevisiae,* the catalase from *Aspergillus niger,* or the like. It is preferred to use the catalase T from *Saccharomyces cerevisiae;* however, it should be further appreciated that for enzymatic conversion of glycolate to glyoxylate in the absence of AMPA any compatible heterologous catalase is contemplated as being useful for purposes of this invention.

The methylotrophic yeast *Hansenula polymorpha* has been developed as an expression system for heterologous proteins (Roggenkamp et al., Mol. Gen. Genetics, (1986), 202: 302; Hollenberg and Janowicz, EPA No. 0299108 (1987)). As a facultative methylotroph this yeast species is able to use methanol as sole energy and carbon source; other possible carbon sources are glucose or glycerol. Upon addition of methanol into culture media, key enzymes of the methanol metabolism are strongly expressed. The expression is regulated at a transcriptional level. The genes for these key enzymes dihydroxyacetone synthase (DHAS), formate dehydrogenase (FMD; i.e., FMDH) and methanol oxidase (MOX) have been cloned and characterized (Ledeboer et al., Nucleic Acids Res., (1985), 13: 3060; Janowicz et al., Nucleic Acids Res., (1985), 13: 2043; Hollenberg and Janowicz, supra (1987)). The promoters of these key enzymes are repressed using glucose, derepressed using glycerol and induced using methanol as carbon source.

The promoter elements of these genes can be used for heterologous gene expression. Accordingly, they are components of expression vectors for the generation of recombinant *Hansenula polymorpha* strains. A standard expression vector harbors the coding sequence of a foreign gene integrated between the promoter, for example but not by way of limitation, an FMD- promoter, a MOX - promoter or the like, and generally any terminator, again by way of example but not by way of limitation, a MOX-terminator sequence or the like. In addition, the vectors contain selection markers and a HARS1 *(Hansenula* autonomously replicating sequence) for selection and propagation in suitable *H. polymorpha* hosts and a bacterial origin of replication (ori) and an ampicillin-resistance (amp) gene for propagation and selection in *E. coli* (Gellissen et al., Biotech Adv., (1992), 10, 179; Gellissen et al., Tibtech, (1992), 10,413).

After uptake, the heterologous DNA is stably integrated into the host's genome. Transformations result in a variety of strains harboring a varying copy number of the integrated DNA in a head-to-tail arrangement. This stable multimeric integration of expression cassettes makes *Hansenula polymorpha* an ideal host for the co-expression of several proteins in a fixed ratio (Janowicz et al., Yeast, (1991), 7: 431).

The construction of an expression cassette for the expression of glycolate oxidase or catalase in *Hansenula* may be accomplished by means well known to those skilled in the art. The source of the glycolate oxidase gene (or the catalase gene) may either be chromosomal DNA or a previously constructed vector containing the gene. Generally, it is most preferred to isolate the glycolate oxidase gene from an already existing vector. It is also preferred that the glycolate oxidase gene be bounded on both the 5' and 3' ends by convenient restrictions sites. Any vector or plasmid containing a suitable glycolate oxidase gene may be used, however, for the purpose of the present invention the plasmid pDA-PCR#1 is most preferred. pDA-PCR#1 is derived from the *Aspergillus* transformation plasmid pTAwtS-GOD. More specifically, pTAwtS-GOD contains a spinach glycolate oxidase gene under the control of a *Aspergillus nidulans alcA* promoter and bounded at the 5' end by a *Bgl*II site and at the 3' end by an *Eco*RI site. The glycolate oxidase gene in pTAwtS-GOD is amplified by conventional PCR protocols using primers which incorporated an *Xba*I site at one end and an *Eco*RI site at the opposite end. The PCR fragment is ligated between the *Xba*I and *Eco*RI sites in the Bluescript plasmid (Stratagene, La Jolla, CA) to give the plasmid pDA-PCR#1.

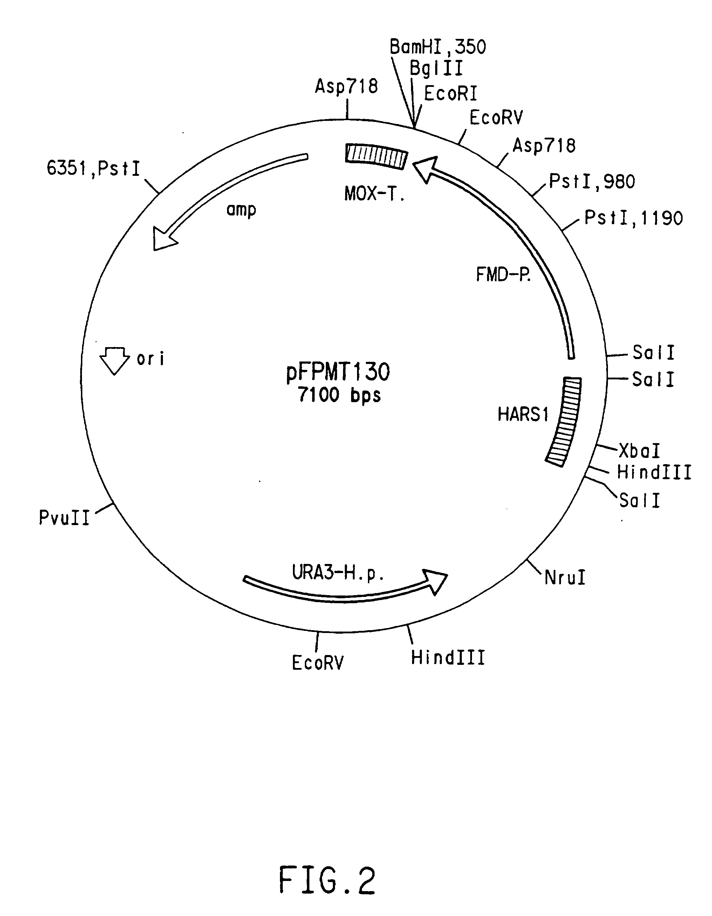

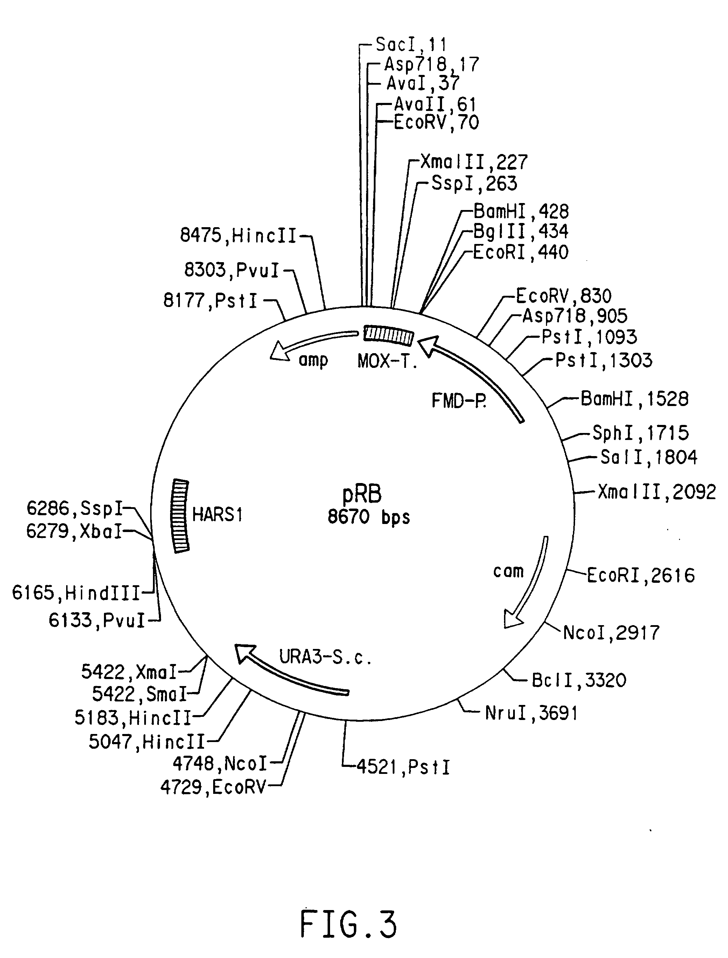

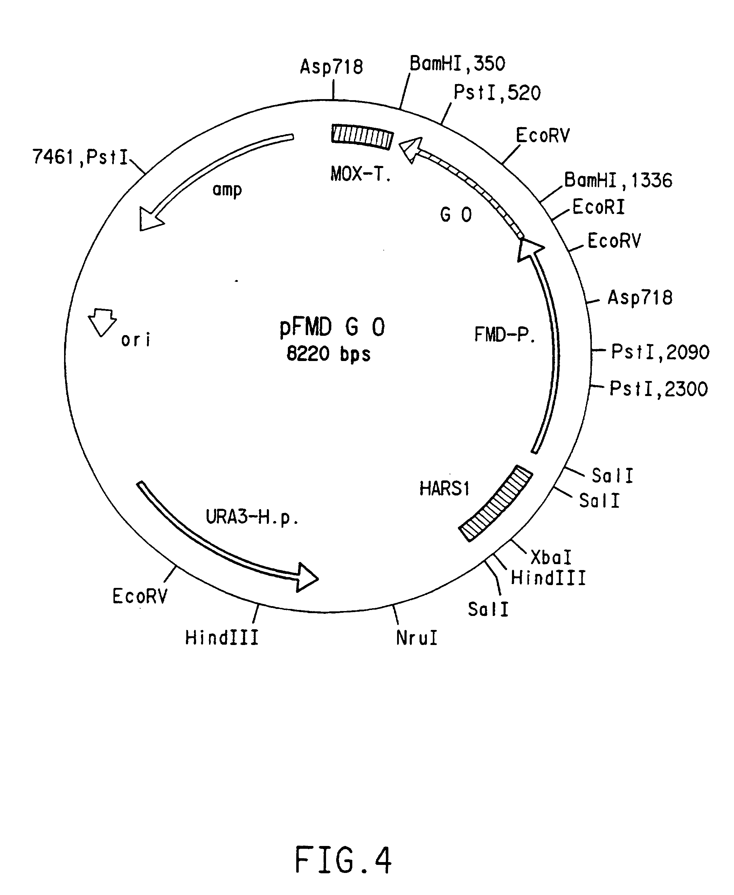

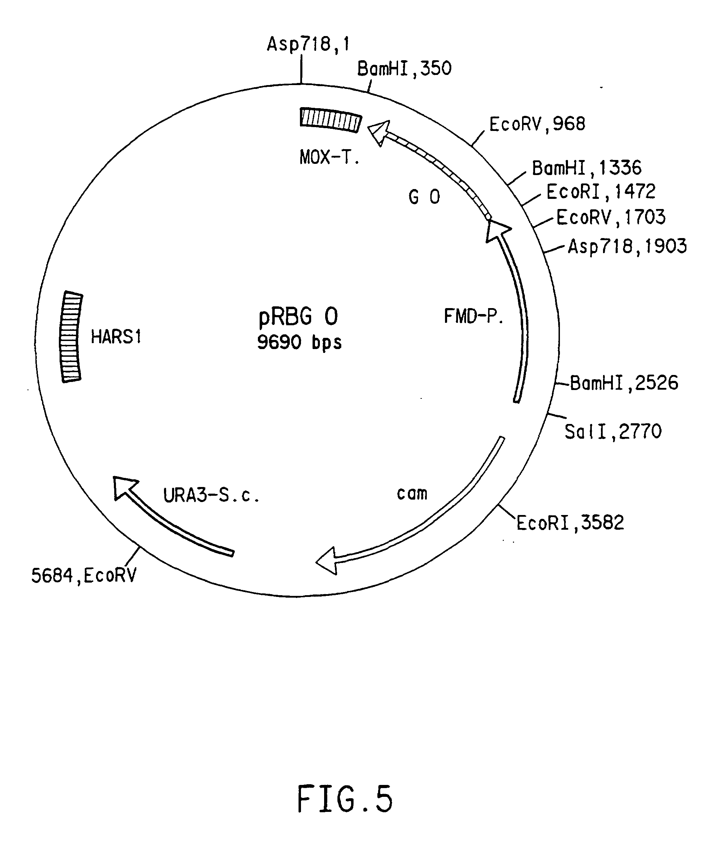

Isolated DNA encoding the glycolate oxidase protein is optionally amplified by techniques well known in the art for the purpose of cloning into a suitable *Hansenula* transformation vector. Any method of amplification may be used including Polymerase Chain Reaction (PCR) or Ligase Chain Reaction (LCR) where PCR is most preferred. Amplified glycolate oxidase DNA is then cloned into a suitable *Hansenula* transformation vector. A number of transformation vectors are suitable where the vector contains a promoter capable of driving the glycolate oxidase gene and where the promoter contains, downstream, a restriction site compatible with the restriction sites flanking the glycolate oxidase gene. Any suitable transformation vector may be used, including pFPMT130 or pRB described in detail in Figures 2 and 3. The restricted fragment is cloned into the multiple cloning site of the basic vector pFPMT130 (see Figure 2) using the *Eco*RI and the *Bam*HI site for insertion. A second series is constructed in the pRB vector harboring a chloramphenicol resistance sequence as a selection marker (Figure 3). Restriction, ligation, propagation, and isolation of the newly generated plasmid DNA follows standard procedures as described by Maniatis et al., Molecular Cloning: a laboratory manual, Cold Spring Harbour Laboratory Press, (1982). The insertion of the cDNA sequence results in the expression vectors pFMDGO and pRBGO (Figures 4 and 5).

Transformation of *H. polymorpha*

The vectors described above are used to transform competent *H. polymorpha* cells of strain RB11, deficient in orotidine 5' phosphate dehydrogenase (ura-). The auxotrophic strain RB 11 is generated basically as described by Roggenkamp et al., *supra,* 1986. Competent cells of this strain are generated according to established protocols (Dohmen et al., Yeast (1991), 7, 691) as follows: 10 mL yeast medium (YPD; i.e., yeast, peptone, and glucose) are inoculated with cells and cultured at 37 °C overnight. This culture is subsequently used to inoculate 200 mL of YPD. Cell are cultured at 37 °C until the $OD_{600}$ is between 0.6 and 1.0. Cells are harvested by centrifugation, washed at room temperature with 100 mL of a solution A (1 M sorbitol, 10 mM bicine pH 8.35, 3% ethylene glycol) and then resuspended in 4 mL of this solution A; 11 µL of dimethylsulfoxide (DMSO) is added and the competent cells are stored at -70 °C.

For transformation, 10 µg of plasmid DNA and 100 µl of cold 0.1 M $CaCl_2$ are added to the frozen cell aliquots; after fast thawing 1.0 mL of a solution B (40% PEG 3350, 200 mM bicine pH 8.35) is added, and the transformation mixtures are incubated at 37 °C for 1 hour. Subsequently, cells are washed in 1 mL of a solution C (150 mM NaCl, 10 mM bicine pH 8.35) and resuspended in 200 mL. This suspension is plated on selective agar plates (yeast nitrogen base (YNB)-glucose). Plates are incubated at 37 °C for 3 to 5 days.

## Generation of mitotically stable strains with multimeric copies of the heterologous DNA

Colonies from developed plates are used to inoculate 3 mL of YNB glucose and cultured at 37 °C. A 50 μL aliquot of the fully grown culture is used to inoculate another 3 mL culture. This procedure is repeated for some 40 generations of growth. During this passaging plasmid DNA is integrated into the genome. Subsequently 3 mL of YPD (non-selective medium) is inoculated and cultured at 37 °C. Plating of a diluted aliquot should result in an identical number of colonies when plated on selective and non-selective agar plates.

## Identification of recombinant strains expressing glycolate oxidase (GO)

For expression of the recombinant GO, the cells have to be cultured under derepressive or inductive conditions. The passaged transformants were used to inoculate 3 mL of YNB supplemented with 1% glycerol. After two days of growth the cells were transferred to 3 mL YNB supplemented with 1% methanol. After a further day of induction cells were harvested by centrifugation (5 min 800 x g) and GO activity was determined in samples prepared from crude extracts.

## Preparation of crude extract

Harvested cells are resuspended in 600 mL of extraction buffer (1 mM dithiothreitol (DTT), 0.1 mM flavin mononucleotide (FMN), 10 mM phenol methylsulfonyl fluoride (PMSF), 10% DMSO in 0.1 M sodium phosphate buffer pH 8.3). Cells are broken with glass beads (0.45-0.5 mm diameter) for 5 minutes, cooling with solid $CO_2$ every 30 seconds. Cell debris is removed by centrifugation (15 minutes at 15000 x g at 4 °C).

## Determination of GO activity

5-20 mL of the crude extracts were analyzed for GO content by quantifying the generation of glyoxylic acid by spectrophotometric assay modified after Soda et al., Agr. Biol. Chem., (1973), 37(6):1393.

## Copy number determination of the integrated heterologous DNA in GO-expressing strains

The copy number of the integrated heterologous DNA is determined as outlined by Gellissen et al., *supra* (1992). For DNA determination, DNA is isolated from various transformants and from the untransformed host strain RB 11. The isolated DNA is restricted with *Asp*718/*Sal*I, transferred to nitrocellulose and hybridized to a $^{32}$P-labeled *Eco*RI/*Asp*718 fragment. This results in two signals in similar electrophoretic positions for the genuine single copy FMD/GO gene fusion and the heterologous FMD promoter/GO gene fusion. In DNA dilutions the copy number is estimated comparing the signal intensity of the heterologous fragments with that of the intrinsic single copy control.

## Properties of GO production strains

Transformed strains were passaged and analyzed for GO content as described. The following screening results were obtained using pFMDGO and pRBGO for transformation.

Glycolate oxidase protein may be detected by Western blot analysis or glycolate oxidase activity may be detected by means of a spectrophotometric assay. Most preferred is the method described by Soda et al., *supra,* (1973). This assay measures the glyoxylate produced by the glycolate oxidase-catalyzed oxidation of glycolate by reacting said glyoxylate with glycine and o-aminobenzaldehyde to form a yellow complex having an absorbance maximum at 440 nm.

The *Hansenula* strains were also evaluated for their ability to produce endogenous catalase. For the evaluation of catalase production transformed *Hansenula polymorpha* RB11, was grown according to the procedure described above and analyzed for enzymatically active catalase (i.e., any disproportionation of hydrogen peroxide as determined by assays of conventional design). Several methods of determining catalase activity are available, such as the method of Beers et al., J. Biol. Chem., (1952), 195:133.

The following examples are presented to more fully demonstrate and further illustrate various individual aspects of the present invention. In doing so, *Hansenula polymorpha* is intentionally employed as the host illustrative of the genus *Hansenula* and similarly certain specific sources of genetic code and sequences are identified for various purposes as being illustrative only. As such, the example are felt to be non-limiting and are meant to illustrate the invention but are not meant to be unduly limiting in any way.

EXAMPLE 1

The GO gene as found in pDA-PCR#1, which is derived from the *Aspergillus* transformation plasmid pTAwt-S-GOD, was excised using *Eco*RI and *Bam*HI. More specifically, pTAwtS-GOD contains a spinach glycolate oxidase gene under the control of a *Aspergillus nidulans alcA* promoter and bounded at the 5' end by a *Bgl*II site and at the 3' end by an *Eco*RI site. The glycolate oxidase gene in pTAwtS-GOD is amplified by conventional PCR protocols using primers which incorporated an *Xba*I site at one end and an *Eco*RI site at the opposite end. The PCR fragment is ligated between the XbaI and EcoRI sites in the Bluescript plasmid (Stratagene, La Jolla, CA) to give the plasmid pDA-PCR#1. For a more detailed description of this procedure see copending and commonly assigned U.S. Patent application serial number USSN 08/085,488.

The restricted fragment was cloned into the multiple cloning site of the basic vector pFPMT130 (see Figure 2) using the *Eco*RI and the *Bam*HI site for insertion. Restriction and ligations, propagation and isolation of the newly generated plasmid DNA followed standard procedures as described by Maniatis et al., *supra,* (1982). The insertion of the cDNA sequence resulted in the expression vectors pFMDGO (Figure 4).

EXAMPLE 2

A second series was constructed in the pRB vector harboring an chloramphenicol resistance sequence as a selection marker (Figure 3). The GO gene as found in pDA-PCR#1 was excised using *Eco*RI and *Bam*HI. The restricted fragment was cloned into the multiple cloning site of the vector pRB (Figure 3) using the *Eco*RI and the *Bam*HI site for insertion. Restriction and ligations, propagation and isolation of the newly generated plasmid DNA followed standard procedures as described by Maniatis et al., *supra,* (1982). The insertion of the cDNA sequence resulted in the expression vector pRBGO (Figure 5).

EXAMPLE 3

Transformation of *H. polymorpha* with pFMDGO

The vector pFMDGO was used to transform competent *H. polymorpha* cells of strain RB11, deficient in ortidin 5' phosphate dehydrogenase (ura⁻). The auxotrophic strain RB 11 was generated basically as described by Roggenkamp et al., *supra,* (1986). Competent cells of this strain are generated according to established protocols (Dohmen et al., *supra,* (1991)) as follows: 10 mL yeast medium (YPD) were inoculated with cells and cultured at 37°C overnight. This culture was subsequently used to inoculate 200 mL of YPD. Cells were grown at 37°C to an $OD_{600}$ of 0.6 to 1.0. Cells were harvested by centrifugation, washed at room temperature in 100 mL of a solution A (1 M sorbitol, 10 mM bicine pH 8.35 3% ethylene glycol) and then resuspended in 4 mL of solution A; 11 μL DMSO. were added and the competent cells were stored at -70 °C.

For transformation 10 μg of plasmid DNA and 100 mL of cold 0.1 M $CaCl_2$ were added to the frozen cell aliquots; after fast thawing 1.0 mL of a solution B (40% PEG 3350, 200 mM bicine pH 8.35) was added, the transformation mixtures was incubated at 37°C for 1 hour. Subsequently cells were washed in 1 mL of a solution C (150 mM NaCl, 10 mM bicine pH 8.35) and resuspended in 200 μL. This suspension was plated on selective agar plates (YNB-glucose). Plates were incubated at 37 °C for 3 to 5 days.

Mitotically stable strains with multimeric copies of the heterologous DNA were generated by passage stabilization. Colonies from developed plates were used to inoculate 3 mL of YNB glucose and cultured at 37 °C. A 50 μL aliquot of the fully grown culture was used to inoculate another 3 mL culture. This procedure was repeated for some 40 generations of growth. During this passaging plasmid DNA was integrated into the genome. Subsequently 3 mL of YPD (non-selective medium) was inoculated and cultured at 37 °C. Diluted aliquots of passage stabilized cells resulted in an identical number of colonies when plated on selective and non-selective agar plates.

The passaged transformants were used to inoculate 3 mL of YNB supplemented with 1% glycerol. After two day of growth the cells were transferred to 3 mL YNB supplemented with 1% methanol. After a further day of induction cells were harvested by centrifugation (5 min. at 800 x g). Harvest cells were resuspended in 600 mL of extraction buffer (1 mM DTT, 0.1 mM FMN, 10 mM PMSF, 10% DMSO in 0.1 sodium phosphate buffer pH 8.3). Cells were broken with glass beads (0.45-0.5 mn diameter for 5 minutes cooling with $CO_2$ every 30 seconds. Cell debris was removed by centrifugation (15 minutes at 15000 x g at 4°C). Glycolate oxidase activity was measured from cell lysates using a spectrophotometric assay (with absorbance monitoring at 440 nm) for GO enzyme activity employing o-aminobenzaldehyde and glycine (Soda et al., 1973). Results are summarized in Table I with activities being reported per mg of protein.

EP 0 706 572 B1

TABLE I

| pFMDGO Transformed RB11 Strain No. | GO activity mU/mg |
|---|---|
| 11.2.01 | 1042 |
| 11.2.02 | 709 |
| 11.2.08 | 772 |
| 11.2.12 | 124 |
| 11.2.14 | 2047 |
| 11.2.16 | 1855 |
| 11.2.17 | 2910 |
| 11.2.18 | 1504 |
| 11.2.21 | 945 |
| 11.2.23 | 467 |
| 11.2.24 | 281 |
| 11.2.26 | 270 |

EXAMPLE 4

Transformation of *H. polymorpha* with pRBGO

The vector pRBDGO was used to transform competent *H. polymorpha* cells of strain RB 11, essentially as described in Example 3. After passage stabilization and assay for glycolate oxidase activity the following clones were selected:

TABLE II

| pRBGO Transformed RB 11 Strain No. | GO activity mU/mg |
|---|---|
| 11.11.80 | 5958 |
| 11.11.89 | 3055 |
| 11.11.95 | 4281 |
| 11.11.109 | 3771 |
| 11.11.126 | 7524 |
| 11.11.165 | 3583 |
| 11.11.177 | 4480 |
| 11.11.178 | 4492 |
| 11.11.179 | 3689 |
| 11.11.180 | 3137 |

One strain, 11.11.126, was selected for further subcloning. The results are shown in Table **III.**

TABLE III

| Subclones of 11. 11. 126 Strain No. | GO activity mU/mg |
|---|---|
| 11.11.126.1 | 4111 |
| 11.11.126.2 | 71 |
| 11.11.126.3 | 3495 |
| 11.11.126.4 | 0 |
| 11.11.126.5 | 2266 |
| 11.11.126.6 | 5092 |

TABLE III (continued)

| Subclones of 11. 11. 126 Strain No. | GO activity mU/mg |
| --- | --- |
| 11.11.126.7* | 7026 |
| 11.11.126.8 | 4187 |
| 11.11.126.9 | 3274 |
| 11.11.126.10 | 4232 |

*The best strain from this subcloning, 11.11.126.7, was selected and designated *Hansenula polymorpha* GO1 and deposited under the terms of the Budapest Treaty with U.S. Department of Agriculture, Northern Regional Research Laboratories, located in Peoria, IL, and is designated by the accession number NRRL No. Y-21065

### EXAMPLE 5

The copy number of the integrated heterologous DNA was determined for *Hansenula polymorpha* GO1 as outlined by Gellissen et al. *(supra,* (1992)). DNA was isolated from the transformant and from the untransformed host strain RB 11. The isolated DNA was restricted with *Asp*718/*Sal*I, transferred to nitrocellulose and hybridized to a $^{32}$P-labeled *Eco*RI/*Asp*718 fragment. This results in two signals in similar electrophoretic positions for the genuine single copy FMD gene and the heterologous FMD promoter/GO fusion. In DNA dilutions the copy number was estimated comparing the signal intensity of the heterologous fragments with that of the intrinsic single copy control. This determination revealed that the *Hansenula polymorpha* GO1 contains approximately 30 copies of the integrated plasmid. This recombinant strain is mitotically stable and remains unchanged during fermentation.

### EXAMPLE 6

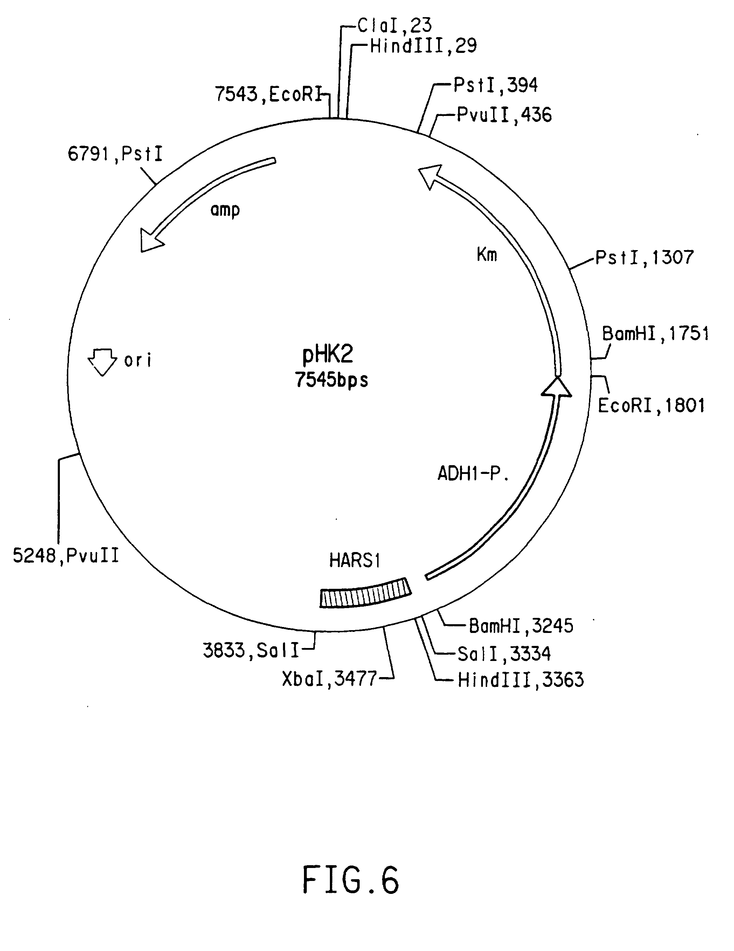

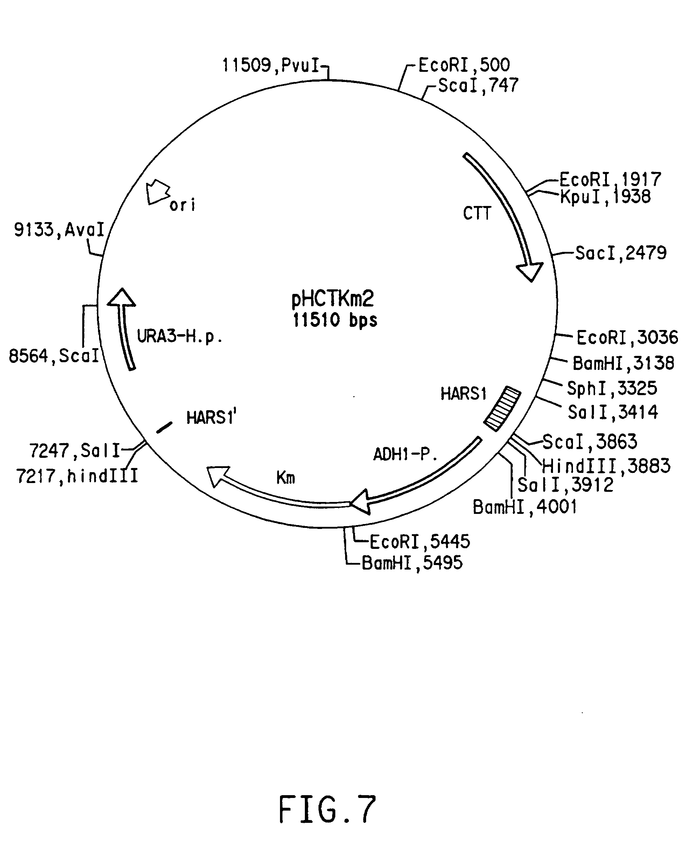

The expression plasmids for CTT1 were designed for super transformation of the GO expressing strain *Hansenula polymorpha* GO1 described in Example 4. The basic expression vectors harbor an ADH1 promoter/kanamycin resistance fusion as an additional dominant selection marker for the identification of supertransformants from a *Hansenula polymorpha* GO1 background. The kanamycin resistance gene is derived from *E. coli* transposon Tn5. The CTT1 gene was obtained from the plasmid pHCT-124 (Hansen and Roggenkamp, *supra* (1989)) harboring the CTT1 coding sequence fused to the genuine CTT1 promoter. Transformation of *H. polymorpha* strains with this plasmid is reported to result in recombinant strains expressing CTT1 (Hansen and Roggenkamp, *supra* (1989)). An ADH1/kanamycin resistance fusion, obtained as a *Hind*III fragment from the plasmid pHK2 (Figure 6) was inserted into the plasmid pHCT-124 resulting in vector pHCTKm2 (Figure 7).

The transformation followed essentially the procedure outlined in Example 3 except cells of the strain *Hansenula polymorpha* GO1 served as the host. For selection of transformants, mixtures were plated on YNB plates containing gentamycin (G418 sulfate; GIBCO, 1.5 mg in 400 µL, applied to the surface of hardened 20 mL agar plates).

### EXAMPLE 7

In a second construction the CTT1 coding sequence from pHCT-124 was subcloned into a M13 vector and mutagenized by site-directed mutagenesis to obtain *Bgl*II restriction sites flanking the ATG start and the TAA stop codon, of the CTT1 gene. After insertion of the CTT1 gene and the ADH1/kanamycin resistance fusion (described in Example 6) into pRB (Figure 3) the resulting vector is designated pRBCATT and is shown in Figure 4. It contains the CTT1 sequence inserted between the *FMD*-promoter and the MOX-terminator sequence as described for the GO expression plasmids in Example 1.

Transformation of *Hansenula polymorpha* GO1 with pRBCATT and identification of strains coexpressing GO and catalase T was essentially as described in Example 6.

Passaged clones coexpressing GO and catalase T activities were identified by culturing on YNB supplemented with glycerol and then with methanol for promoter induction as described before.

The total catalase activity (catalase T plus endogenous *H. polymorpha* catalase) was determined by preparing extracts of the double transformants and assaying the extracts of 2-5 mg of wet cells into a 3-mL quartz cuvette containing a magnetic stirring bar, then adding 2.0 mL of 16.7 mM phosphate buffer (pH 7.0) followed by 1.0 mL of 59 mM hydrogen peroxide in 16.7 mM phosphate buffer (pH 7.0) and stirring the suspension while measuring the change in absorption with time at 240 nm ($\varepsilon$ = 39.4). The catalase T and endogenous catalase activities of microbial double transformants were each determined by comparison to the activity determined by this procedure at pH 4.0; at pH 4.0, the endogenous *H. polymorpha* catalase retains 7% of its activity at pH 7.0, while the Catalase T retains 60% of its activity at pH 7.0.

The catalase T activity can also be estimated in crude extracts by its lower mobility relative to *H. polymorpha* catalase on native polyacrylamide gel electrophoresis. Crude extracts from methanol-induced cells prepared as described in Example 3 were separated on 7.5% native gels in the Phastgel system (Pharmacia, Sweden). After separation gels were incubated with gentle shaking in $H_2O_2$ (100 μL 30% $H_2O_2$ in 100 mL water) for 10 minutes. The solution was discarded and the gels were then briefly rinsed in water. Then the gels were incubated with gentle shaking in "Prussian Blue" solution (freshly prepared 1:1 mixture of 2% $FeCl_3$ ($Fe(CN)_6$)). Catalase activity is indicated by a clear zone on a blue background. The relative size of the clear zone is related to the catalase activity. When using plasmid pRBCATT 76 clones with varying expression levels of CTT1 in addition to the GO expression in terms of enzyme activity per mg of protein could be identified (Tables IV and V).

TABLE IV

| Hansenula polymorpha DOUBLE TRANSFORMANTS | | | | | |
|---|---|---|---|---|---|
| Transformant** | GO (U/mg) | catalase total (U/mg) | catalase T (U/mg) | % catalase T | Copy (#) |
| 7.13.3 | 3.95 | 2780 | 706 | 25 | 2 |
| 7.13.24 | 3.39 | 2710 | 873 | 32 | 10-12 |
| 7.13.168 | 4.23 | 2647 | 873 | 33 | 10-12 |
| 7.13.171 | 3.30 | 2064 | 412 | 20 | <2 |
| 7.13.175 | 4.32 | 1202 | 436 | 36 | 10-12 |

**Strains 7.13.168 and 7.13.3 from this subcloning were selected and designated *Hansenula polymorpha* GO2 and GO3, respectively, and deposited under the terms of the Budapest Treaty with U.S. Department of Agriculture, Northern Regional Research Laboratories, located in Peoria, IL, on June 25, 1993 and are designated by the accession numbers NRRL No. Y-21113 and Y-21114.

TABLE V

| GO and Catalase T Expression of Individualized Clones at a 3 mL Scale | | | | | | |
|---|---|---|---|---|---|---|
| Number | Strain Number | GO (U/mg) | catalase total (U/mg) | catalase T (U/mg) | % catalase T | Protein (mg/mL) |
| 1 | 7.13.7.1 | 1.3 | 1058 | / | / | 0.13 |
| 2 | 7.13.7.2 | 2.4 | 2117 | / | / | 0.07 |
| 3 | 7.13.7.3 | 2.6 | 1814 | / | / | 0.07 |
| 4 | 7.13.7.4 | 2.2 | 1653 | / | / | 0.09 |
| 5 | 7.13.7.5 | 1.8 | 1628 | / | / | 0.13 |
| 6 | 7.13.7.6 | 4.5 | 3705 | 3008 | / | 0.04 |
| 7 | 7.13.44.1 | 2.2 | 2084 | 536 | 26 | 0.32 |
| 8 | 7.13.44.2 | 1.1 | 1861 | 30 | 2 | 0.29 |
| 9 | 7.13.44.3 | 1.6 | 1745 | 525 | 30 | 0.37 |
| 10 | 7.13.44.4 | 2.7 | 1562 | 839 | 54 | 0.21 |
| 11 | 7.13.44.5 | 2.2 | 1600 | 67 | 4 | 0.43 |
| 12 | 7.13.44.6 | 1.6 | 1190 | 93 | 8 | 0.48 |
| 13 | 7.13.44.7 | 2.3 | 2531 | 1538 | 61 | 0.736 |
| 14 | 7.13.44.8 | 4.0 | 2487 | 1080 | 43 | 0.52 |
| 15 | 7.13.44.9 | 4.6 | 4969 | 2194 | 44 | 0.392 |
| 16 | 7.13.44.10 | 5.7 | 3269 | 1358 | 42 | |
| 17 | 7.13.45.1 | 4.5 | 7410 | / | / | 0.02 |
| 18 | 7.13.45.2 | 1.9 | 818 | / | / | 0.44 |
| 19 | 7.13.45.3 | 3.9 | 2587 | 1736 | 67 | 0.356 |
| 20 | 7.13.56.1 | 1.5 | 2550 | 299 | 12 | 0.22 |
| 21 | 7.13.56.2 | 2.3 | 1672 | 515 | 31 | 0.38 |

TABLE V   (continued)

| GO and Catalase T Expression of Individualized Clones at a 3 mL Scale | | | | | | |
|---|---|---|---|---|---|---|
| Number | Strain Number | GO (U/mg) | catalase total (U/mg) | catalase T (U/mg) | % catalase T | Protein (mg/mL) |
| 22 | 7.13.56.3 | 2.3 | 2079 | 1255 | 60 | 0.35 |
| 23 | 7.13.61.1 | 2.3 | 2069 | 942 | 45 | 0.46 |
| 24 | 7.13.61.2 | 1.1 | 2588 | 324 | 13 | 0.18 |
| 25 | 7.13.63.6 | 1.8 | 1482 | 1002 | 68 | 0.05 |
| 26 | 7.13.63.7 | 5.1 | 3148 | 459 | 15 | |
| 27 | 7.13.63.8 | 5.9 | 4333 | 2561 | 59 | 0.408 |
| 28 | 7.13.72.3 | 3.0 | 1726 | 508 | 29 | 0.19 |
| 29 | 7.13.72.6 | 1.7 | 1989 | 222 | 11 | 0.33 |
| 30 | 7.13.145.1 | 5.1 | 2969 | 933 | 31 | 0.61 |
| 31 | 7.13.145.3 | 3.3 | 814 | 405 | 50 | 0.39 |
| 33 | 7.13.150.4 | 2.5 | 2487 | 1020 | 41 | 0.45 |
| 34 | 7.13.150.6 | 2.6 | 876 | 160 | 18 | 0.29 |
| 35 | 7.13.155 | 3.1 | 3350 | 1456 | 43 | 0.452 |
| 36 | 7.13.155.3 | 4.3 | 1651 | 493 | 30 | 0.59 |
| 37 | 7.13.155.8 | 4.4 | 3448 | 1695 | 49 | 0.344 |
| 38 | 7.13.155.1 | 4.2 | 4337 | 1831 | 42 | 0.332 |

EXAMPLE 8

Strains with promising levels of catalase T were analyzed for copy number of the integrated CTT1 expression cassette and corresponding copy numbers are presented in Table VI. The copy number determination follows closely the procedure described for copy number determination of the GO expression cassette. DNA from various isolates was restricted with *Asp*718, transferred to nitrocellulose and hybridized to the FMD-promoter probe described in Example 5. An additional signal originating from the heterologous FMD-promoter/CTT1 fusion appears and its signal intensity can be compared with that of the 30 copy *FMD*-promoter/GO fusion determined for the host.

TABLE VI

| Number | Strain | Copy Number |
|---|---|---|
| 1 | 7.13.7.6 | 3 |
| 2 | 7.13.44.7 | 15 |
| 3 | 7.13.44.9 | 25 |
| 4 | 7.13.45.3 | 10 |
| 5 | 7.13.56.2 | 20 |
| 6 | 7.13.61.1 | 12 |
| 7 | 7.13.63.6 | 1 |
| 8 | 7.13.63.8 | 2 |
| 9 | 7.13.72.3 | 15 |
| 10 | 7.13.145.1 | 2 |
| 11 | 7.13.145.3 | 2 |
| 12 | 7.13.155.3 | 7 |
| 13 | 7.13.155.8 | 7 |
| 14 | 7.13.155.12 | 7 |

Transformants of *Hansenula* hosts which co-express glycolate oxidase and catalase according to the instant invention are useful for the manufacture of glyoxylic acid from glycolic acid (hydroxyacetic acid). They are also useful as catalysts for the enzymatic conversion of mixtures of glycolic acid and AMPA to N-(phosphonomethyl)glycine, a post-emergent phytotoxicant and herbicide as well as a source for the enzymes, *per se.*

## EP 0 706 572 B1

**Claims**

1. A *Hansenula* transformant comprising heterologous DNA encoding a heterologous glycolate oxidase or an enzymatically active variant thereof which is capable of converting glycolate to glyoxylate wherein the heterologous DNA comprises coding regions operably linked to suitable regulatory sequence elements, wherein the suitable regulatory sequence elements comprise a formate dehydrogenase (FMD) promoter and a methanol oxidase (MOX) terminator sequence, and further wherein the heterologous DNA are stably integrated within a host chromosome.

2. The *Hansenula* strain of claim 1 further comprising heterologous DNA encoding a heterologous exogenous catalase or an enzymatically active variant thereof which catalyses the decomposition of hydrogen peroxide, wherein the heterologous DNA comprises coding regions operably linked to suitable regulatory sequence elements, and further wherein the heterologous DNA are stably integrated within the host chromosome.

3. The *Hansenula* strain of claim 1, wherein said heterologous DNA encodes for spinach glycolate oxidase or an enzymatically active variant thereof which is capable of converting glycolate to glyoxylate.

4. The *Hansenula* transformant according to claim 3, wherein said host is a *Hansenula* species selected from the group consisting of *Hansenula polymorpha*, *Hansenula augusta*, *Hansenula anomala*, *Hansenula capsulata*, *Hansenula saturnus,* and *Hansenula wingei.*

5. The *Hansenula* transformant according to claim 3, wherein said host is of the species *Hansenula polymorpha* and said heterologous DNA encodes for spinach glycolate oxidase.

6. A microorganism *Hansenula polymorpha* (NRRL Y-21065).

**7.** A microorganism *Hansenula polymorpha* (NRRL Y-21113).

**8.** A microorganism *Hansenula polymorpha* (NRRL Y-21114).

**9.** The *Hansenula* transformant according to claim 2, wherein said exogenous catalase is not inhibited by aminomethylphosphonic acid.

**10.** The *Hansenula* transformant according to claim 9, wherein said exogenous catalase is derived from *Saccharomyces cerevisiae* catalase T or *Aspergillus niger.*

**11.** A method for producing enzymatically active glycolate oxidase, comprising the step of culturing a *Hansenula* transformant comprising heterologous DNA encoding a heterologous glycolate oxidase or an enzymatically active variant thereof which is capable converting glycolate to glyoxylate wherein the heterologous DNA comprises. coding regions operably linked to suitable regulatory sequence elements, wherein the suitable regulatory sequence elements comprise a formate dehydrogenase (FMD) promoter and a methanol oxidase (MOX) terminator sequence, and further wherein the heterologous DNA is stably integrated within a host chromosome.

**12.** A method for producing enzymatically active glycolate oxidase and catalase, comprising the step of culturing a *Hansenula* transformant comprising heterologous DNA encoding a heterologous glycolate oxidase or an enzymatically active variant thereof which is capable of converting glycolate to glyoxylate and DNA encoding an exogenous catalase or an enzymatically active variant thereof which catalyses the decomposition of hydrogen peroxide wherein the heterologous DNA comprises coding regions operably linked to suitable regulatory sequence elements, wherein the suitable regulatory sequence elements comprise a formate dehydrogenase (FMD) promoter and a methanol oxidase (MOX) terminator sequence, and further wherein the heterologous DNA is stably integrated within a host chromosome.

**13.** A method as claimed in claim 11 wherein the *Hansenula* transformant additionally comprises a heterologous DNA encoding an exogenous catalase or an enzymatically active variant thereof which catalyses the decomposition of hydrogen peroxide wherein the heterologous DNA comprises coding regions operably linked to suitable regulatory sequence elements, and further wherein the heterologous DNA is stably integrated within a host chromosome.

**Patentansprüche**

**1.** *Hansenula*-Transformante, die heterologe DNA, für eine heterologe Glycolatoxidase oder eine ihrer enzymatisch aktiven Varianten, die Glycolat in Glyoxylat umwandeln kann, codiert, enthält, wobei die heterologe DNA Codierregionen enthält, die operativ mit geeigneten Regulatorsequenzelementen verknüpft sind, wobei die geeigneten Regulatorsequenzelemente eine Formiatdehydrogenase (FMD)-Promotor- und eine Methanoloxidase (MOX)-Terminatorsequenz enthalten und wobei weiterhin die heterologe DNA stabil in ein Wirtschromosom integriert ist.

**2.** *Hansenula*-Stamm nach Anspruch 1, der weiterhin heterologe DNA, die für eine heterologe exogene Katalase oder eine ihrer enzymatisch aktiven Varianten, die den Zerfall von Wasserstoffperoxid katalysiert, codiert, enthält, wobei die heterologe DNA Codierregionen, die operativ mit geeigneten Regulatorsequenzelementen verknüpft sind, umfaßt und wobei weiterhin die heterologe DNA stabil in dem Wirtschromosom integriert ist.

**3.** *Hansenula*-Stamm nach Anspruch 1, wobei die heterologe DNA für eine Spinat-Glycolatoxidase oder eine ihrer enzymatisch aktiven Varianten, die Glycolat in Glyoxylat umwandeln kann, codiert.

**4.** *Hansenula*-Transformante nach Anspruch 3, wobei es sich bei dem Wirt um eine *Hansenula*-Art aus der Reihe *Hansenula-polymorpha, Hansenula augusta*, *Hansenula anomala, Hansenula capsulata, Hansenula saturnus* und *Hansenula wingei* handelt.

**5.** *Hansenula*-Transformante nach Anspruch 3, wobei der Wirt zu der Art *Hansenula polymorpha* zählt und die heterologe DNA für Spinat-Glycolatoxidase codiert.

**6.** Mikroorganismus *Hansenula polymorpha* (NRRL Y-21065).

**7.** Mikroorganismus *Hansenula polymorpha* (NRRL Y-21113)*.*

**8.** Mikroorganismus *Hansenula polymorpha* (NRRL Y-21114).

**9.** *Hansenula*-Transformante nach Anspruch 2, wobei die exogene Katalase nicht durch Aminomethylphosphonsäure inhibiert wird.

**10.** *Hansenula*-Transformante nach Anspruch 9, wobei die exogene Katalase von *Saccharomyces cerevisiae* Katalase T oder *Aspergillus niger* stammt.

**11.** Verfahren zur Herstellung einer enzymatisch aktiven Glycolatoxidase, das einen Schritt umfaßt, bei dem man eine *Hansenula*-Transformante, die heterologe DNA, für eine heterologe Glycolatoxidase oder eine ihrer enzymatisch aktiven Varianten, die Glycolat in Glyoxylat umwandeln kann, codiert, enthält, wobei die heterologe DNA Codierregionen enthält, die operativ mit geeigneten Regulatorsequenzelementen verknüpft sind, wobei die geeigneten Regulatorsequenzelemente eine Formiatdehydrogenase (FMD)-Promotor- und eine Methanoloxidase (MOX)-Terminatorsequenz enthalten und wobei weiterhin die heterologe DNA stabil in ein Wirtschromosom integriert ist, kultiviert.

**12.** Verfahren zur Herstellung von enzymatisch aktiver Glycolatoxidase und Katalase, das einen Schritt umfaßt, bei dem man eine *Hansenula*-Transformante, die heterologe DNA, für eine heterologe Glycolatoxidase oder eine ihrer enzymatisch aktiven Varianten codiert, die Glycolat in Glyoxylat umwandeln kann und DNA, die für eine exogene Katalase oder eine ihrer enzymatisch aktiven Varianten, die den Zerfall von Wasserstoffperoxid katalysiert, codiert, wobei die heterologe DNA Codierregionen enthält, die operativ mit geeigneten Regulatorsequenzelementen verknüpft sind, wobei die geeigneten Regulatorsequenzelemente eine Formiatdehydrogenase (FMD)-Promotor- und eine Methanoloxidase (MOX)-Terminatorsequenz enthalten und wobei weiterhin die heterologe DNA stabil in ein Wirtschromosom integriert ist, kultiviert.

**13.** Verfahren nach Anspruch 11, wobei die *Hansenula*-Transformante zusätzlich eine heterologe DNA, die für eine exogene Katalase oder eine ihrer enzymatisch aktiven Varianten, die den Zerfall von Wasserstoffperoxid katalysiert, codiert, enthält, wobei die heterologe DNA Codierregionen, die operativ mit geeigneten Regulatorsequenzelementen verknüpft sind, umfaßt und wobei weiterhin die heterologe DNA stabil in dem Wirtschromosom integriert ist.

**Revendications**

**1.** Transformant *d'Hansenula* comprenant des ADN hétérologues codant pour une glycolate oxydase hétérologue ou son variant actif enzymatiquement, qui est apte à transformer du glycolate en glyoxylate, les ADN hétérologues comprenant des régions de codage reliées fonctionnellement à des éléments de séquence appropriés de régulation et les éléments de séquence appropriés de régulation comprennent une séquence de promoteur de formiate désydrogénase (FMD) et une séquence de terminateur de méthanol oxydase (MOX), et dans lequel en outre les ADN hétérologues sont intégrés de manière stable au sein d'un chromosome hôte.

**2.** Souche d'*Hansenula* suivant la revendication 1, comprenant en outre des ADN hétérologues codant pour une catalase hétérologue exogène ou son variant actif enzymatiquement, qui catalyse la décomposition du peroxyde d'hydrogène, l'ADN hétérologue comprenant des régions de codage reliées fonctionnellement à des éléments de séquence appropriés de régulation, et en outre dans laquelle les ADN hétérologues sont intégrés de manière stable au sein du chromosome hôte.

**3.** Souche d'*Hansenula* suivant la revendication 1, dans laquelle l'ADN hétérologue code pour la glycolate oxydase de l'épinard ou son variant actif enzymatiquement, qui est apte à transformer du glycolate en du glyoxylate.

**4.** Transformant d'*Hansenula* suivant la revendication 3, dans lequel l'hôte est une espèce *Hansenula* choisie dans le groupe consistant en *Hansenula polymorpha, Hansenula augusta, Hansenula anomala, Hansenula capsulata, Hansenula saturnus et Hansenula wingei.*

**5.** Transformant d'*Hansenula* suivant la revendication 3, dans lequel l'hôte est de l'espèce *Hansenula polymorpha* et l'ADN hétérologue code pour la glycolate oxydase de l'épinard.

**6.** Un microorganisme *Hansenula polymorpha* (NRRL Y-21065).

**7.** Un microorganisme *Hansenula polymorpha* (NRRL Y-21113).

**8.** Un microorganisme *Hansenula polymorpha* (NRRL Y-21114).

**9.** Transformant d'*Hansenula* suivant la revendication 2, dans lequel la catalase exogène n'est pas inhibée par l'acide aminométhylphosphonique.

**10.** Transformant d'*Hansenula* suivant la revendication 9, dans lequel la catalase exogène provient de la *Saccharomyces cerevisiae catalase* T ou d'*Aspergillus niger.*

**11.** Procédé de préparation d'une glycolate oxydase active enzymatiquement, comprenant le stade de culture d'un transformant d'*Hansenula* comprenant des ADN hétérologues codant pour une glycolate oxydase hétérologue ou son variant actif enzymatiquement, qui est apte à transformer du glycolate en du glyoxylate, l'ADN hétérologue comprenant des régions de codage reliées fonctionnellement à des éléments de séquence appropriés de régulation, et les éléments de séquence appropriés de régulation comprennent une séquence de promoteur de formiate désydrogénase (FMD) et une séquence de terminateur de méthanol oxydase (MOX), et dans lequel en outre les ADN hétérologues sont intégrés de manière stable au sein d'un chromosome hôte.

**12.** Procédé de préparation de glycolate oxydase et de catalase active enzymatiquement, comprenant le stade de culture d'un transformant d'*Hansenula* comprenant de l'ADN hétérologue codant pour une glycolate oxydase hétérologue ou son variant actif enzymatiquement, qui est apte à transformer du glycolate en du glyoxylate et de l'ADN codant pour une catalase exogène ou son variant actif enzymatiquement, qui catalyse la décomposition du peroxyde d'hydrogène, l'ADN hétérologue comprenant des régions de codage reliées fonctionnellement à des éléments de séquence appropriés de régulation, et les éléments de séquence appropriés de régulation comprennent une séquence de promoteur de formiate désydrogénase (FMD) et une séquence de terminateur de méthanol oxydase (MOX), et dans lequel en outre les ADN hétérologues sont intégrés de manière stable au sein d'un chromosome hôte.

**13.** Procédé suivant la revendication 11, dans lequel le transformant d'*Hansenula* comprend en outre un ADN hétérologue codant pour une catalase exogène ou son variant actif enzymatiquement, qui catalyse la décomposition du peroxyde d'hydrogène, l'ADN hétérologue comprenant des régions de codage reliées fonctionnellement à des éléments de séquence appropriés de régulation, et l'ADN hétérologue étant en outre intégré de manière stable au sein d'un chromosome hôte.

| 1   | MEITNVNEYE | AIAKQKLPKM | VYDYYASGAE | DQWTLAENRN | AFSRILFRPR |
|-----|------------|------------|------------|------------|------------|
| 51  | ILIDVTNIDM | TTTILGFKIS | MPIMIAPTAM | QKMAHPEGEY | ATARAASAAG |
| 101 | TIMTLSSWAT | SSVEEVASTG | PGIRFFQLYV | YKDRNVVAQL | VRRAERAGFK |
| 151 | AIALTVDTPR | LGRREADIKN | RFVLPPFLTL | KNFEGIDLGK | MDKANDSGLS |
| 201 | SYVAGQIDRS | LSWKDVAWLQ | TITSLPILVK | GVITAEDARL | AVQHGAAGH  |
| 251 | VSNHGARQLD | YVPATIMALE | EVVKAAQGRI | PVFLDGGVRR | GTDVFKALAL |
| 301 | GAAGVFIGRP | VVFSLAAEGE | AGVKKVLQMM | RDEFELTMAL | SGCRSLKEIS |
| 351 | RSHIAADWDG | PSSRAVARL  |            |            | Seq. No. 1 |

FIG.1

EP 0 706 572 B1

FIG.2

FIG.3

FIG.4

FIG.5

FIG.6

FIG.7

FIG.8